# EUROPÄISCHE PATENTSCHRIFT

(11) **EP 3 448 321 B1**
(45) Veröffentlichungstag und Bekanntmachung des Hinweises auf die Patenterteilung: **17.03.2021**
(21) Anmeldenummer: 17719248.1
(22) Anmeldetag: 25.04.2017
(51) Int. Cl.: A61F 2/72, A61F 2/54, A61F 2/60, A61F 2/76

(54) **PROTHESE**
PROSTHESIS
PROTHÈSE

(30) Priorität: 25.04.2016 DE 102016107615
(43) Veröffentlichungstag der Anmeldung: 06.03.2019
(73) Patentinhaber: Cavos Bagatelle Verwaltungs GmbH & Co. KG, 80538 München (DE)
(72) Erfinder: HADDADIN, Sami, 30173 Hannover (DE)
(74) Vertreter: Rösler Rasch van der Heide & Partner
(86) Internationale Anmeldenummer: PCT/EP2017/059699
(87) Internationale Veröffentlichungsnummer: WO 2017/186659

(56) Entgegenhaltungen:
- DE-A1-102009 056 466
- US-A1- 2012 221 119
- US-A1- 2014 336 781
- US-A1- 2015 257 903

## Beschreibung

Die Erfindung betrifft eine Prothese zum Ersatz einer fehlenden Extremität eines Lebewesens, insbesondere eines Menschen. Die Prothese weist mittels Aktoren angetriebene Prothesenglieder auf.

Heute verfügbare aktorisch angetriebene Prothesen, beispielsweise Hand-Arm-Prothesen sind in ihrer Anschaffung mit erheblichen Kosten verbunden. Darüber hinaus sind diese Prothesen in ihrer Funktion eingeschränkt.

Eine Prothese mit einem adaptiven Steuerungs- und Regelungssystem ist zum Beispiel in der Druckschrift DE 10 2009 056466 A1 bekannt. Andere Prothesen sind in US 2014/336781 A1 und US 2012/221119 A1 offenbart.

Die Aufgabe der Erfindung ist es, eine verbesserte Prothese zum Ersatz einer fehlenden Extremität eines Lebewesens, insbesondere eines Menschen bereitzustellen, die kostengünstig herstellbar ist und über eine erweiterte Funktionalität verfügt.

Die Erfindung ergibt sich aus den Merkmalen des unabhängigen Anspruchs. Vorteilhafte Weiterbildungen und Ausgestaltungen sind Gegenstand der abhängigen Ansprüche. Weitere Merkmale, Anwendungsmöglichkeiten und Vorteile der Erfindung ergeben sich aus der nachfolgenden Beschreibung, sowie der Erläuterung von Ausführungsbeispielen der Erfindung, die in den Figuren dargestellt sind.

Die Aufgabe ist mit gelöst mit einer Prothese zum Ersatz einer fehlenden Extremität eines Lebewesens, insbesondere eines Menschen. Die vorgeschlagene Prothese umfasst ein oder mehrere mittels Aktoren angetriebene Prothesenglieder, wobei das proximale Prothesenglied eine mechanische Schnittstelle zum Fixieren des proximalen Prothesenglieds an dem Lebewesen aufweist.

Die Prothese umfasst weiterhin erste Sensoren, die einen aktuellen Zustand ZUS(t) der Prothese, insbesondere einen Kontaktzustand der Prothese mit einer Umgebung erfassen. Vorteilhaft umfassen die ersten Sensoren eine oder mehrere der folgenden Sensoren: Gelenkssensor/en zur Erfassung einer Motorposition und/oder einer Motorgeschwindigkeit und/oder einer Motorbeschleunigung, Abtriebssensor/en zur Erfassung einer Abtriebsposition und/oder einer Abtriebsgeschwindigkeit und/oder einer Abtriebsbeschleunigung, Beschleunigungssensor/en, beispielsweise zur Erfassung einer Beschleunigung in den Gelenken der Prothese oder einer Beschleunigung in den Prothesengliedern, der Erdbeschleunigung, einer Beschleunigung und Bewegung der mechanische Schnittstelle zum Fixieren des proximalen Prothesenmitglieds, einer Relativbeschleunigung und Bewegung der Prothese auf dem Gliedstumpf, Kraftsensor/en zur Ermittlung der durch die einzelnen Prothesenglieder auf eine Umgebung übertragene Kräfte und zur Ermittlung von einer Umgebung auf die einzelnen Prothesenglieder übertragenen Kräfte sowie zur Ermittlung von Interaktionskräften zwischen Prothese und Prothesenträger, Drehmomentsensor/en zur Ermittlung der durch die einzelnen Prothesenglieder auf eine Umgebung übertragene Drehmomente und zur Ermittlung von einer Umgebung auf die einzelnen Prothesenglieder übertragenen Drehmomente, taktile/r Sensor/en, insbesondere eine künstliche Haut, zur lokalen Erfassung von einwirkenden Kräften und Momenten, Temperatursensor/en zur Erfassung von auf die Prothese einwirkenden Temperaturen, Feuchtesensor/en zur Erfassung einer auf die Prothese einwirkenden Feuchte. Alle Sensoren (aber auch einzelne) können vorteilhaft auch genutzt werden, um mechanische Relationen (Kräfte, Beschleunigungen etc.) zu ermitteln.

Die Prothese umfasst weiterhin eine Schnittstelle zu zweiten Sensoren, die Biosignale SIG_{BIO}(t) des Lebewesens zur Steuerung der fehlenden Extremität erfassen. Die zweiten Sensoren umfassen vorteilhaft einen oder mehrere Elektromyographie-Sensoren und/oder einen oder mehrere Elektroenzephalographie-Sensoren. Die einzelnen zweiten Sensoren sind vorteilhaft entweder auf dem Lebewesen appliziert oder in das Lebewesen implantiert.

Die Prothese umfasst weiterhin dritte Sensoren zur Erfassung von Daten D_{UMG}(t), die eine aktuelle Umgebung der Prothese, insbesondere in der Umgebung befindliche Objekte und/oder anderen Lebewesen beschreiben. Die dritten Sensoren können einen oder mehrere der folgenden Sensoren umfassen: optische Sensoren, die beispielsweise Kamera- oder Video-Sensoren, Ultraschallsensoren, Lasersensoren, etc.

Die Prothese umfasst weiterhin eine Prädiktionseinheit, die auf Basis der Biosignale SIG_{BIO}(t) und des Zustands ZUS(t) der Prothese und der Daten D_{UMG}(t) ein Modell M_{A}(t) einer mit der Prothese auszuführenden Aktion A ermittelt und von dem Modell M_{A}(t) abhängige Bewegungen B_{eweg}(M_{A}(t)) der Prothesenglieder für einen Zeitraum [t, t+Δt] prädiziert. Im einfachsten Fall erfolgt die Tradition für einen nächsten Zeitschritt. Dieser kann der oder die Regltakte der Prothese sein. Der Prädiktionszeitraum Δt wird vorteilhaft im Bereich von 0.3 bis 30 Sekunden gewählt. Der Prädiktionszeitraum Δt kann vorteilhaft variieren je nach Prozessorauslastung in der Prädiktionseinheit. Vorteilhaft ist die Prädiktionseinheit derart ausgeführt und eingerichtet, dass die Ermittlung des Modells M_{A}(t) bzw. M_{A}(t) als lernfähiger Prozess implementiert ist, der autonom basierend auf historischen Daten zum Ausführen einer Aktion A/A' hierfür prädizierten Bewegungen B_{eweg}(M_{A}(t))/B_{eweg}(M_{A'}(t)) der Prothesenglieder lernt. Diese können auch prädizierte/geschätzte Kontakte und/oder andere multimodale Information berücksichtigen.

Die Prothese umfasst weiterhin eine Bewertungseinheit mit der auf Basis einer Bewertung der Biosignale SIG_{BIO}(t), des Zustands ZUS(t), der Daten D_{UMG}(t) und der prädizierten Bewegungen B_{eweg}(M_{A}(t)) nach einem vorgegebenen Bewertungsschema die diskrete Entscheidung E ermittelbar ist, die Aktion A durch eine andere Aktion A'(E) zu ersetzen, wobei die Aktion A'(E) eine reflexive und/oder protektive autonom gesteuerte/geregelte Bewegung der Prothese definieren kann, und wobei sofern die Aktion A'(E) keine solche reflexive und/oder protektive autonom gesteuerte/geregelte Bewegung der Prothese definiert, durch die Prädiktionseinheit ein Modell M_{A}(t) der mit der Prothese auszuführenden Aktion A' ermittelt wird, und von dem Modell M_{A}(t) abhängige Bewegungen B_{eweg}(M_{A}(t)) der Prothesenglieder für einen Zeitraum [t, t+Δt] prädiziert werden.

Die Bewertungseinheit ist vorteilhaft derart ausgeführt und eingerichtet, dass eine Entscheidung E für eine Aktion A'(E), die eine reflexive und/oder protektive autonome Bewegung der Prothese definiert unabhängig von den Biosignalen SIG_{BIO}(t) auf Basis des aktuellen Zustands ZUS(t) und/oder der Daten D_{UMG}(t) ermittelt wird.

Die Bewertungseinheit ist vorteilhaft derart ausgeführt und eingerichtet, dass eine Entscheidung E für eine Aktion A'(E), die eine reflexive und/oder protektive autonome Bewegung der Prothese definiert, dann getriggert wird, wenn
- eine aktuelle Bewegung der Prothesenglieder von der prädizierten Bewegung B_{eweg}(M(t)) oder B_{eweg}(M_{A'}(t)) der Prothesenglieder mehr als ein vorgegebener Grenzwert abweicht (dieser Grenzwert kann zeitvariante und/oder abhängig vom Zustand ZUS(t) sein), und/oder
- eine aktuelle Bewegung der Prothesenglieder von einem gelerntem Modell, das Nominalzustände der Prothese und entsprechende kritischen Abweichungen von den Nominalzuständen beobachtet, abweicht, und/oder
- auf Basis des aktuellen Zustands ZUS(t) und der Daten D_{UMG}(t) erkennbar ist, dass die mit der Prothese auszuführende Aktion A oder A'(E) nicht oder fehlerhaft ausgeführt wurde oder ausgeführt wird (dadurch kann beispielsweise ein Nachfassen bei einem fehlerhaften Ergreifen eines Objekts durch die Prothese erfolgen), und/oder
- eine unbeabsichtigte Kollision eines oder mehrerer Prothesenglieder mit einem Objekt in der Umgebung erfolgt ist oder erfolgen wird, und/oder
- eine von einem Temperatursensor der Prothese erfasste Temperatur einen Grenzwert erreicht oder überschreitet, und/oder
- ein distales Ende der Prothese sich einem Objekt bis auf eine vorgegebene Distanz oder Umgebungshülle genähert hat (dies dient insbesondere zum Ausführen von sogenannten "Standard Skills", wie beispielsweise das (teil-)autonome Greifen eines Objekts, das Bewegen eines Schalters eines Tasters, Öffnen einer Türe, das Ergreifen und zum Mund Führen einer Bierflasche, etc.), und/oder
- der aktuelle Zustand ZUS(t) der Prothese einem Zustand entspricht, für den gilt: ZUS(t) ∉ **Z**_{ZUS,erlaubt}, wobei **Z**_{ZUS,erlaubt} die Menge aller erlaubten Zustände ZUS(t) angibt (ZUS(t) können auch abgeleitete Größen sein. So können beliebige Mannigfaltigkeiten, Beboachter, Modelle mit Methoden des Machinellen Lernens gelernt sein).

Die Prothese umfasst schließlich eine Steuereinheit, die auf Basis der aktuell gültigen prädizierten Bewegungen B_{eweg}(M_{A}(t)) oder B_{eweg}(M_{A'}(t)) oder auf Basis der reflexiven und/oder protektiven autonom gesteuerten/geregelten Bewegung Steuersignale Sig(t) zur Steuerung der Aktoren ableitet und die Aktoren auf Basis der Steuersignale Sig(t) steuert/regelt.

Die vorgeschlagene Prothese ermöglicht durch die Auswertung der SIG_{BIO}(t), durch die Nutzung der angeführten Sensoren zur Erfassung des aktuellen Zustands ZUS(t) der Prothese wie auch eines Zustands einer Umgebung der Prothese und durch die Modellbildung und Prädiktion eine verbesserte und natürlichere Steuerung der Prothese, wobei die Prothese in der Lage ist, autonome Bewegungen und Aktionen, insbesondere reflexive und/oder protektive autonome Bewegung auszuführen.

Eine vorteilhafte Weiterbildung der Prothese zeichnet sich dadurch aus, dass die Steuereinheit eine Eingabeschnittstelle aufweist, über die ein Nutzer der Prothese durch eine Eingabe die Steuereinheit veranlasst, jedwede Bewegung der Prothese zu stoppen. Dies ermöglicht es dem Träger der Prothese jederzeit eine Aktivität der Prothese zu stoppen.

Eine vorteilhafte Weiterbildung der Prothese zeichnet sich dadurch aus, dass die Steuereinheit derart ausgeführt und eingerichtet ist, dass nach einer Ausführung einer reflexiven und/oder protektiven autonomen Bewegung eine weitere Bewegung der Prothese auf Basis aktuell erfasster Biosignalen SIG_{BIO}(t) und/oder nach einer Eingabe eines Nutzers der Prothese über eine Schnittstelle der Steuereinheit erfolgt, wobei die Schnittstelle zur manuellen optischen und/oder akustischen und/oder taktilen Eingabe ausgeführt und eingerichtet ist. Vorteilhaft wird die Steuereinheit aus mehreren multimodalen Steuereinheiten gebildet. Weiterhin vorteilhaft ist die Steuereinheit derart ausgeführt und eingerichtet, dass die Steuerung der Aktoren eine Drehmomentregelung mit einer Reibungskompensation und/oder eine Impedanz- und/oder eine Kraftregelung aufweist.

Eine vorteilhafte Weiterbildung der Prothese zeichnet sich dadurch aus, dass die Steuereinheit zur Steuerung der Prothese mehrere Apps (Steuerprogramme und Steuerparmetersätze) bereitstellt, wobei jede App einen Betriebsmodus der Prothese definiert, und wobei die Steuereinheit eine Schnittstelle, insbesondere eine drahtlose Schnittstelle, zu einer mobilen Einheit aufweist, über die eine jeweilige App zur Steuerung der Prothese anwählbar und konfigurierbar ist. Die mobile Einheit ist vorteilhaft ein Notebook oder ein Smartphone oder ein mobiler Tablet-Computer.

Eine vorteilhafte Weiterbildung der Prothese zeichnet sich dadurch aus, dass die Steuereinheit derart ausgeführt und eingerichtet ist, dass auf Basis des aktuellen Zustands ZUS(t) und der Daten D_{UMG}(t) eine automatische Anpassung der mechanischen aktiven und oder passiven Impedanz, insbesondere der Steifigkeit der Prothese durch eine entsprechende Änderung der Steuersignale Sig(t) erfolgt.

Die vorgeschlagene Prothese ist vorteilhaft als eine Handprothese, eine Unterarmprothese mit und ohne Ellbogen, eine Vollarmprothese, eine Beinprothese, oder eine Prothese mit einem Exoskelett ausgeführt. Bei der vorgeschlagenen Prothese können ein, mehrere oder alle Prothesenglieder mittels Aktoren, d.h. aktorisch angetrieben sein.

Eine vorteilhafte Weiterbildung der Prothese zeichnet sich dadurch aus, dass eine Rückmeldeschnittstelle vorhanden ist, über die dem Prothesenträger Rückmeldungen über den aktuellen Zustand ZUS(t) der Prothese und deren Interaktion mit der Umgebung übermittelt werden. Diese Rückmeldung erfolgt bevorzugt haptisch oder durch elektrische Stimulation.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist. Gleiche, ähnliche und/oder funktionsgleiche Teile sind mit gleichen Bezugszeichen versehen.

Es zeigt:

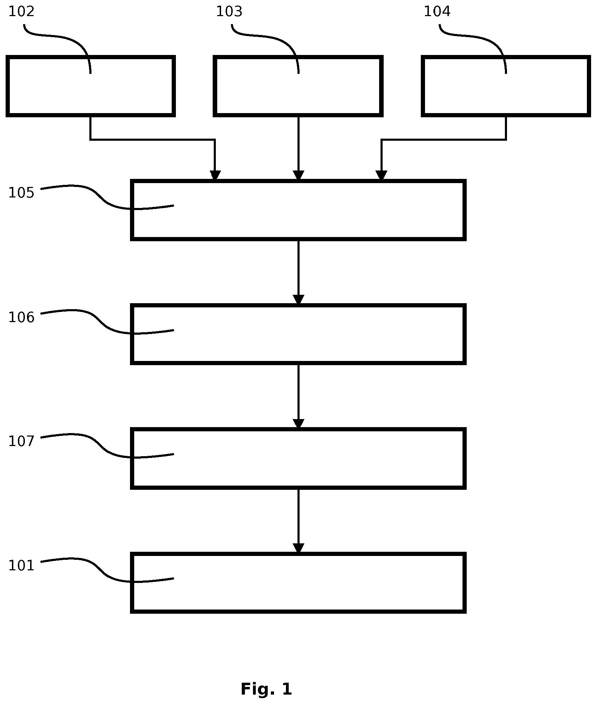

- Fig. 1: einen schematisierten Aufbau einer vorgeschlagenen Prothese.

Fig. 1 zeigt einen schematisierten Aufbau einer vorgeschlagenen Prothese zum Ersatz einer fehlenden Extremität eines Lebewesens, insbesondere eines Menschen, die aufweist: ein oder mehrere mittels Aktoren **101** angetriebene Prothesenglieder, wobei das proximale Prothesenglied eine mechanische Schnittstelle zum Fixieren des proximalen Prothesenglieds an dem Lebewesen aufweist, erste Sensoren **102,** die einen aktuellen Zustand ZUS(t) der Prothese, insbesondere einen Kontaktzustand der Prothese mit einer Umgebung erfassen, eine Schnittstelle zu zweiten Sensoren **103,** die Biosignale SIG_{BIO}(t) des Lebewesens zur Steuerung der fehlenden Extremität erfassen, dritte Sensoren **104** zur Erfassung von Daten D_{UMG}(t), die eine aktuelle Umgebung der Prothese, insbesondere in der Umgebung befindliche Objekte und/oder anderen Lebewesen beschreiben, eine Prädiktionseinheit **105,** die auf Basis der Biosignale SIG_{BIO}(t) und des Zustands ZUS(t) der Prothese und der Daten D_{UMG}(t) ein Modell M_{A}(t) einer mit der Prothese auszuführenden Aktion A ermittelt und von dem Modell M_{A}(t) abhängige Bewegungen B_{eweg}(M_{A}(t)) der Prothesenglieder für einen Zeitraum [t, t+Δt] prädiziert, eine Bewertungseinheit **106** mit der auf Basis einer Bewertung der Biosignale SIG_{BIO}(t), des Zustands ZUS(t), der Daten D_{UMG}(t) und der prädizierten Bewegungen B_{eweg}(M_{A}(t)) nach einem vorgegebenen Bewertungsschema die diskrete Entscheidung E ermittelbar ist, die Aktion A durch eine andere Aktion A'(E) zu ersetzen, wobei die Aktion A'(E) eine reflexive und/oder protektive autonom gesteuerte/geregelte Bewegung der Prothese definieren kann, und wobei sofern die Aktion A'(E) keine solche reflexive und/oder protektive autonom gesteuerte/geregelte Bewegung der Prothese definiert, durch die Prädiktionseinheit **105** ein Modell M_{A}(t) der mit der Prothese auszuführenden Aktion A' ermittelt wird, und von dem Modell M_{A}(t) abhängige Bewegungen B_{eweg}(M_{A'}(t)) der Prothesenglieder für einen Zeitraum [t, t+Δt] prädiziert werden, und eine Steuereinheit 107, die auf Basis der aktuell gültigen prädizierten Bewegungen B_{eweg}(M_{A}(t)) oder B_{eweg}(M_{A'}(t)) oder auf Basis der reflexiven und/oder protektiven autonom gesteuerten/geregelten Bewegung Steuersignale Sig(t) zur Steuerung der Aktoren ableitet und die Aktoren auf Basis der Steuersignale Sig(t) steuert/regelt.

Obwohl die Erfindung im Detail durch bevorzugte Ausführungsbeispiele näher illustriert und erläutert wurde, so ist die Erfindung nicht durch die offenbarten Beispiele eingeschränkt und andere Variationen können vom Fachmann hieraus abgeleitet werden, ohne den Schutzumfang der Erfindung zu verlassen. Es ist daher klar, dass eine Vielzahl von Variationsmöglichkeiten existiert. Es ist ebenfalls klar, dass beispielhaft genannte Ausführungsformen wirklich nur Beispiele darstellen, die nicht in irgendeiner Weise als Begrenzung etwa des Schutzbereichs, der Anwendungsmöglichkeiten oder der Konfiguration der Erfindung aufzufassen sind. Vielmehr versetzen die vorhergehende Beschreibung und die Figurenbeschreibung den Fachmann in die Lage, die beispielhaften Ausführungsformen konkret umzusetzen, wobei der Fachmann in Kenntnis des offenbarten Erfindungsgedankens vielfältige Änderungen beispielsweise hinsichtlich der Funktion oder der Anordnung einzelner, in einer beispielhaften Ausführungsform genannter Elemente vornehmen kann, ohne den Schutzbereich zu verlassen, der durch die Ansprüche und deren rechtliche Entsprechungen, wie etwa weitergehenden Erläuterungen in der Beschreibung, definiert wird.

### Bezugszeichenliste

- 101: Aktoren
- 102: erste Sensoren
- 103: zweite Sensoren
- 104: dritte Sensoren
- 105: Prädiktionseinheit
- 106: Bewertungseinheit
- 107: Steuereinheit

## Patentansprüche

1. Prothese zum Ersatz einer fehlenden Extremität eines Lebewesens, insbesondere eines Menschen, die aufweist:
- ein oder mehrere mittels Aktoren (101) angetriebene Prothesenglieder, wobei das proximale Prothesenglied eine mechanische Schnittstelle zum Fixieren des proximalen Prothesenglieds an dem Lebewesen aufweist,
- eine Schnittstelle zu zweiten Sensoren (103), die Biosignale SIG_{BIO}(t) des Lebewesens zur Steuerung der fehlenden Extremität erfassen,
- dritte Sensoren (104) zur Erfassung von Daten D_{UMG}(t), die eine aktuelle Umgebung der Prothese, insbesondere in der Umgebung befindliche Objekte und/oder anderen Lebewesen beschreiben,
**dadurch gekennzeichnet, dass** die Prothese zusätzlich aufweist:
- erste Sensoren (102), die einen aktuellen Zustand ZUS(t) der Prothese, insbesondere einen Kontaktzustand der Prothese mit einer Umgebung erfassen,
- eine Prädiktionseinheit (105), die auf Basis der Biosignale SIG_{BIO}(t) und des Zustands ZUS(t) der Prothese und der Daten D_{UMG}(t) ein Modell M_{A}(t) einer mit der Prothese auszuführenden Aktion A ermittelt und von dem Modell M_{A}(t) abhängige Bewegungen B_{eweg}(M_{A}(t)) der Prothesenglieder für einen Zeitraum [t, t+Δt] prädiziert,
- eine Bewertungseinheit (106) mit der auf Basis einer Bewertung der Biosignale SIG_{BIO}(t), des Zustands ZUS(t), der Daten D_{UMG}(t) und der prädizierten Bewegungen B_{eweg}(M_{A}(t)) nach einem vorgegebenen Bewertungsschema die diskrete Entscheidung E ermittelbar ist, die Aktion A durch eine andere Aktion A'(E) zu ersetzen, wobei die Aktion A'(E) eine reflexive und/oder protektive autonom gesteuerte/geregelte Bewegung der Prothese definieren kann, und wobei sofern die Aktion A'(E) keine solche reflexive und/oder protektive autonom gesteuerte/geregelte Bewegung der Prothese definiert, durch die Prädiktionseinheit (105) ein Modell M_{A}(t) der mit der Prothese auszuführenden Aktion A' ermittelt wird, und von dem Modell M_{A}(t) abhängige Bewegungen B_{eweg}(M_{A'}(t)) der Prothesenglieder für einen Zeitraum [t, t+Δt] prädiziert werden, und
- eine Steuereinheit (107), die auf Basis der aktuell gültigen prädizierten Bewegungen B_{eweg}(M_{A}(t)) oder B_{eweg}(M_{A}(t)) oder auf Basis der reflexiven und/oder protektiven autonom gesteuerten/geregelten Bewegung Steuersignale Sig(t) zur Steuerung der Aktoren ableitet und die Aktoren auf Basis der Steuersignale Sig(t) steuert/regelt.

2. Prothese nach Anspruch 1,
bei der die Steuereinheit (107) eine Eingabeschnittstelle aufweist, über die ein Nutzer der Prothese durch eine Eingabe die Steuereinheit (107) veranlasst, jedwede Bewegung der Prothese zu stoppen.

3. Prothese nach Anspruch 1 oder 2,
bei der die Bewertungseinheit (106) derart ausgeführt und eingerichtet ist, dass eine Entscheidung E für eine Aktion A'(E), die eine reflexive und/oder protektive autonome Bewegung der Prothese definiert unabhängig von den Biosignalen SIG_{BIO}(t) auf Basis des aktuellen Zustands ZUS(t) und/oder der Daten D_{UMG}(t) ermittelt wird.

4. Prothese nach einem der Ansprüche 1 bis 3,
bei der die Bewertungseinheit (106) derart ausgeführt und eingerichtet ist, dass eine Entscheidung E für eine Aktion A'(E), die eine reflexive und/oder protektive autonome Bewegung der Prothese definiert, dann getriggert wird, wenn
- eine aktuelle Bewegung der Prothesenglieder von der prädizierten Bewegung B_{eweg}(M(t)) oder B_{eweg}(M_{A'}(t)) der Prothesenglieder mehr als ein vorgegebener Grenzwert abweicht, und/oder
- eine aktuelle Bewegung der Prothesenglieder von einem gelerntem Modell, das Nominalzustände der Prothese und entsprechende kritischen Abweichungen von den Nominalzuständen beobachtet, abweicht, und/oder
- auf Basis des aktuellen Zustands ZUS(t) und der Daten D_{UMG}(t) erkennbar ist, dass die mit der Prothese auszuführende Aktion A oder A'(E) nicht oder fehlerhaft ausgeführt wurde oder ausgeführt wird, und/oder
- eine unbeabsichtigte Kollision eines oder mehrerer Prothesenglieder mit einem Objekt in der Umgebung erfolgt ist oder erfolgen wird, und/oder
- eine von einem Temperatursensor der Prothese erfasste Temperatur einen Grenzwert G2 erreicht oder überschreitet, und/oder
- ein distales Ende der Prothese sich einem Objekt bis auf eine vorgegebene Distanz oder Umgebungshülle genähert hat, und/oder
- der aktuelle Zustand ZUS(t) der Prothese einem Zustand entspricht, für den gilt: ZUS(t) ∉ **Z**_{zus,erlaubt}, wobei **Z**_{ZUS,erlaubt} die Menge aller erlaubten Zustände ZUS(t) angibt.

5. Prothese nach einem der Ansprüche 1 bis 4,
bei der die Steuereinheit (107) derart ausgeführt und eingerichtet ist, dass nach einer Ausführung einer reflexiven und/oder protektiven autonomen Bewegung eine weitere Bewegung der Prothese auf Basis aktuell erfasster Biosignalen SIG_{BIO}(t) und/oder nach einer Eingabe eines Nutzers der Prothese über eine Schnittstelle der Steuereinheit erfolgt, wobei die Schnittstelle zur manuellen optischen und/oder akustischen und/oder taktilen Eingabe ausgeführt und eingerichtet ist.

6. Prothese nach einem der Ansprühe 1 bis 5,
bei der die ersten Sensoren (102) eine oder mehrere der folgenden Sensoren umfassen:
- Gelenkssensoren zur Erfassung einer Motorposition, Motorgeschwindigkeit und/oder Motorbeschleunigung,
- Abtriebssensoren,
- Beschleunigungssensoren,
- Kraftsensoren zur Ermittlung der durch die einzelnen Prothesenglieder auf eine Umgebung übertragene Kräfte und zur Ermittlung von einer Umgebung auf die einzelnen Prothesenglieder übertragenen Kräfte sowie zur Ermittlung von Interaktionskräften zwischen Prothese und Prothesenträger,
- Drehmomentsensoren zur Ermittlung der durch die einzelnen Prothesenglieder auf eine Umgebung übertragene Drehmomente und zur Ermittlung von einer Umgebung auf die einzelnen Prothesenglieder übertragenen Drehmomente,
- taktile Sensoren, insbesondere eine künstliche Haut, zur lokalen Erfassung von einwirkenden Kräften und Momenten,
- Temperatursensoren zur Erfassung von auf die Prothese einwirkenden Temperaturen,
- feuchte Sensoren zur Erfassung eine auf die Prothese einwirkenden Feuchte.

7. Prothese nach einem der Ansprüche 1 bis 6,
bei der die Steuereinheit (107) zur Steuerung der Prothese mehrere Apps, d.h. Steuerprogramme und -parametersätze, bereitstellt, wobei jede App einen Betriebsmodus der Prothese definiert, und wobei die Steuereinheit eine Schnittstelle, insbesondere eine drahtlose Schnittstelle, zu einer mobilen Einheit aufweist, über die eine jeweilige App zur Steuerung der Prothese anwählbar und konfigurierbar ist.

8. Prothese nach einem der Ansprüche 1 bis 7,
bei der die zweiten Sensoren (103) einen oder mehrere Elektromyographie-Sensoren und/oder einen oder mehrere Elektroenzephalographie-Sensoren umfassen, wobei die einzelnen Sensoren auf dem Lebewesen appliziert oder in das Lebewesen implantiert werden können.

9. Prothese nach einem der Ansprüche 1 bis 8,
bei der die Steuereinheit (107) derart ausgeführt und eingerichtet ist, dass auf Basis des aktuellen Zustands ZUS(t) und der Daten D_{UMG}(t) eine automatische Anpassung der mechanischen aktiven und oder passiven Impedanz, insbesondere der Steifigkeit der Prothese durch eine entsprechende Änderung der Steuersignale Sig(t) erfolgt.

10. Prothese nach einem der Ansprüche 1 bis 9,
bei der die Prädiktionseinheit (105) derart ausgeführt und eingerichtet ist, dass die Ermittlung des Modells M_{A}(t)/M_{A}(t) als lernfähiger Prozess implementiert ist, der autonom basierend auf historischen Daten zum Ausführen einer Aktion A/A' hierfür prädizierten Bewegungen B_{eweg}(M_{A}(t))/ B_{eweg}(M_{A'}(t)) der Prothesenglieder lernt.

## Claims

1. A prosthesis for replacing a missing extremity of a living being, in particular a human being, which prosthesis has:
- one or more prosthesis members driven by means of actuators (101), wherein the proximal prosthesis member has a mechanical interface for attaching the proximal prosthesis member to the living being,
- an interface to second sensors (103), which sense biosignals SIG_{BIO} (t) of the living being for controlling the missing extremity,
- third sensors (104) for capturing data D_{UMG}(t) that describe a current environment of the prosthesis, in particular objects and/or other living beings located in the area surrounding the prosthesis,
**characterized in that** the prosthesis further has:
- first sensors (102), which capture a current state ZUS(t) of the prosthesis, in particular a contact state of the prosthesis with an environment,
- a prediction unit (105) which determines a model MA(t) for an action A to be performed by means of the prosthesis on the basis of the biosignals SIG_{BIO}(t) and the state ZUS(t) of the prosthesis and the data D_{UMG} (t) , and predicts motions B_{EWEG} (M_{A}(t) ) of the prosthesis members for a time period [t, t+Δt], which motions are dependent on the model M_{A}(t),
- an evaluating unit (106), by means of which the discrete decision E to replace the action A with another action A'(E) can be made on the basis of an evaluation of the biosignals SIG_{BIO}(t), the state ZUS(t), the data D_{UMG}(t) and the predicted motions B_{eweg}(M_{A} (t) ) in accordance with a predefined evaluation scheme, wherein the action A'(E) can define a reflexive and/or protective motion of the prosthesis that is autonomously controlled in an open-loop/closed-loop manner, and wherein, if the action A'(E) does not define such a reflexive and/or protective motion of the prosthesis that is autonomously controlled in an open-loop/closed-loop manner, the prediction unit (105) calculates a model M_{A'}(t) of the action A' to be performed by means of the prosthesis and predicts motions B_{eweg}(M_{A'}(t)) of the prosthesis members for a time period [t, t+Δt] depending on the model M_{A'}(t), and
- a control unit (107), which derives control signals Sig(t) for controlling the actuators on the basis of the currently valid predicted motions B_{eweg}(M_{A}(t)) or B_{eweg}(M_{A'}(t)) or on the basis of the reflexive and/or protective motion autonomously controlled in an open-loop/closed-loop manner and controls the actuators in an open-loop/closed-loop manner on the basis of the control signals Sig(t).

2. The prosthesis according to Claim 1,
in which the control unit (107) has an input interface, via which a user of the prosthesis causes the control unit (107) to stop any movement of the prosthesis by making an input.

3. The prosthesis according to Claim 1 or 2,
in which the evaluation unit (106) is designed and configured in such manner that a decision E for an action A'(E) defining a reflexive and/or protective autonomous motion of the prosthesis is calculated on the basis of the current state ZUS(t) and/or the data D_{UMG} (t), independently of the biosignals SIG_{BIO}(t) .

4. The prosthesis according to any one of Claims 1 to 3,
in which the evaluation unit (106) is designed and configured in such manner that a decision E for an action A'(E) defining a reflexive and/or protective autonomous motion of the prosthesis is triggered when
- a current movement of the prosthesis members deviates from the predicted movement B_{eweg}(M(t)) or B_{eweg} (M_{A'}(t)) of the prosthesis members by more than a predetermined limit value, and/or
- a current movement of the prosthesis members deviates from a learned model which observes nominal states of the prosthesis and corresponding critical deviations from the nominal states, and/or
- it is evident on the basis of the current state ZUS(t) and the data D_{UMG}(t) that the action A or A'(E) to be performed with the prosthesis was not performed or is being performed incorrectly, and/ or
- an unintended collision between one or more prosthesis members and an object in the surrounding area has taken place or will take place, and/or
- a temperature detected by a temperature sensor of the prosthesis reaches or exceeds a limit value G2, and/or
- a distal end of the prosthesis has approached an object up to a predetermined distance or surrounding envelope, and/or
- the current state ZUS(t) of the prosthesis corresponds to a state for which the following is true: ZUS(t) ∉ Z_{ZUS.allowed}, where Z_{ZUS.allowed} specifies the set of all allowed states ZUS(t).

5. The prosthesis according to any one of Claims 1 to 4,
in which the control unit (107) is designed and configured in such manner that after a performance of a reflexive and/or protective autonomous movement a further movement of the prosthesis is carried out on the basis of currently captured biosignals SIG_{BIO}(t) and/or after an input by a user of the prosthesis via an interface of the control unit, wherein the interface is designed and configured for manual optical and/or acoustic and/or tactile input.

6. The prosthesis according to any one of Claims 1 to 5,
in which the first sensors (102) comprise one or more of the following sensors:
- joint sensors for capturing a motor position, motor speed and/or motor acceleration,
- output sensors,
- acceleration sensors,
- force sensors for determining the forces transmitted to an environment by the individual prosthesis members and for determining forces transmitted to the individual prosthesis members from the environment, and for determining interaction forces between the prosthesis and the prosthesis wearer,
- torque sensors for determining the torques transmitted to an environment by the individual prosthesis members and for determining torques transmitted to the individual prosthesis members from the environment,
- tactile sensors, in particular an artificial skin, for the local capture of influencing forces and moments,
- temperature sensors for capturing temperatures acting on the prosthesis,
- moisture sensors for capturing a humidity acting on the prosthesis.

7. The prosthesis according to any one of Claims 1 to 6,
in which the control unit (107) provides a plurality of apps, i.e. control programs and parameter sets, for controlling the prosthesis, wherein each app defines an operating mode of the prosthesis, and wherein the control unit has an interface, in particular a wireless interface, with a mobile unit, via which a respective app for controlling the prosthesis can be selected and configured.

8. The prosthesis according to any one of Claims 1 to 7,
in which the second sensors (103) comprise one or more electromyography sensors and/or one or more electroencephalography sensors, wherein the individual sensors are can be attached to the living being or implanted in the living being.

9. The prosthesis according to any one of Claims 1 to 8,
in which the control unit (107) is designed and configured in such manner that an automatic adaptation of the mechanical active and/or passive impedance, in particular the rigidity of the prosthesis based on the current state ZUS(t) and the data D_{UMG}(t) is effected by a corresponding change in the control signals Sig(t).

10. The prosthesis according to any one of Claims 1 to 9,
in which the prediction unit (105) is designed and configured in such manner that the determination of the model M_{A}(t)/M_{A}(t) is implemented as a teachable process which autonomously learns movements B_{eweg}(M_{A}(t)/ B_{eweg} (M_{A'}(t) ) for performing an action A/A' for the prosthetic members based on historical data therefor.

## Revendications

1. Prothèse destinée à remplacer une extrémité manquante d'un être vivant, en particulier d'un être humain, et qui présente :
- ou plusieurs éléments de prothèse entraînés au moyen d'un actionneur (101), l'élément de prothèse proximal présentant une interface mécanique pour la fixation de l'élément de prothèse proximal à l'être humain,
- une interface avec des deuxièmes capteurs (103) qui détectent des signaux biologiques SIGBIO(t) de l'être humain pour commander l'extrémité manquante,
- des troisièmes capteurs (104) pour détecter des données D_{UMG}(t) qui décrivent l'environnement actuel de la prothèse, en particulier des objets se trouvant dans l'environnement et/ou d'autres êtres vivants,
**caractérisée en ce que** la prothèse présente en outre :
- des premiers capteurs (102) qui détectent un état actuel ZUS(t) de la prothèse, en particulier un contact de la prothèse avec un environnement,
- une unité prédictive (105) qui, sur la base des signaux biologiques SIG_{BIO}(t) et de l'état ZUS(t) de la prothèse et des données D_{UMG}(t), détermine un modèle MA(t) d'une action A à réaliser avec la prothèse et prédit des mouvements B_{eweg}(M_{A}(t)) des éléments de la prothèse dépendant du modèle M_{A}(t) pour une période [t, t+Δ],
- une unité de mouvement (106) grâce à laquelle, sur la base d'une évaluation des signaux biologiques SIG_{BIO}(t), de l'état ZUS(t), des données D_{UMG}(t) et des mouvements prédits B_{eweg} (M_{A}(t)) , suivant un schéma d'évaluation 32, la décision discrète E de remplacer l'action A par une autre action A'(E) est déterminable, l'action A'(E) pouvant déterminer un mouvement réflexe et/ ou protecteur commandé/régulé de manière autonome de la prothèse et, dans la mesure où l'action A'(E) ne définit pas un tel mouvement réflexe et/ ou protecteur commandé/régulé de manière autonome de la prothèse, un modèle M_{A'}(t) de l'action A' à réaliser avec la prothèse est déterminé par l'unité prédictive (105), et les mouvements B_{eweg}(M_{A'}(t) ) dépendant du modèle M_{A'}(t) des éléments de la prothèse sont prédits pour une période [t, t+Δ],
- une unité de commande (107) qui, sur la base des mouvements B_{eweg}(M_{A}(t)) ou B_{eweg}(M_{A'}(t)) actuellement en cours ou sur la base du mouvement réflexe et/ou protecteur commandé/régulé de manière autonome, dérive des signaux de commande Sig(t) pour la commande des actionneurs et commande/régule les actionneurs sur la base des signaux de commande Sig(t).

2. Prothèse selon la revendication 1,
dans laquelle l'unité de commande (107) présente une interface de saisie par laquelle un utilisateur de la prothèse, par une saisie, amène l'unité de commande (107) à stopper tout mouvement de la prothèse.

3. Prothèse selon la revendication 1 ou 2,
dans laquelle l'unité d'évaluation (106) est réalisée et conçue de manière à ce qu'une décision E pour une action A' (E) qui définit un mouvement réflexe et/ou protecteur autonome de la prothèse soit déterminée indépendamment des signaux biologiques SIG_{BIO}(t) sur la base de l'état actuel ZUS(t) et/ou des données D_{UMG} (t) .

4. Prothèse selon une des revendications 1 à 3,
dans laquelle l'unité d'évaluation (106) est réalisée et conçue de manière à ce qu'une décision E pour une action A' (E) qui définit un mouvement réflexe et/ou protecteur autonome de la prothèse soit déclenchée si
- un mouvement actuel des éléments de la prothèse diverge du mouvement prédit B_{eweg} (M(t)) ou B_{eweg} (M_{A'}(t)) des éléments de la prothèse plus qu'à raison d'une valeur limite prédéfinie, et/ou
- un mouvement actuel des éléments de la prothèse diverge d'un modèle appris qui observe des états nominaux de la prothèse et des écarts critiques 32 par rapport aux états nominaux, et/ou
- sur la base de l'état actuel ZUS(t) et des données D_{UMG}(t), il apparaît que l'action A ou A' (E) à réaliser avec la prothèse n'a pas ou n'est pas exécutée ou est ou a été mal exécutée, et/ou
- une collision intempestive d'un ou plusieurs éléments de la prothèse avec un objet dans l'environnement a lieu ou va avoir lieu, et/ou
- une température détectée par un capteur de température de la prothèse atteint ou dépasse une valeur limite G2, et/ou
- une extrémité distale de la prothèse s'est approchée d'un objet jusqu'à une distance prédéfinie ou une enveloppe environnante, et/ou
- l'état actuel ZUS(t) de la prothèse correspond à un état pour lequel on a : ZUS(t) ∉ZUS_{Zus.permis}, ZUS_{Zus.permis} indiquant la quantité de tous les étapes permis ZUS(t).

5. Prothèse selon une des revendications 1 à 4,
dans laquelle l'unité de commande (107) est réalisée et conçue de manière à ce que, après une réalisation d'un mouvement réflexe et/ou protecteur autonome, un autre mouvement de la prothèse ait lieu sur la base de signaux biologiques SIG_{BIO}(t) actuellement détectés et/ ou après une saisie d'un utilisateur de la prothèse par l'intermédiaire d'une interface de l'unité de commande, l'interface étant réalisée et conçue pour une saisie optique et/ou acoustique et/ou tactile manuelle.

6. Prothèse selon une des revendications 1 à 5,
dans laquelle les premiers capteurs (102) comprennent un ou plusieurs des capteurs suivants :
- des capteurs d'articulation pour la détection d'une position du moteur, d'une vitesse du moteur/ou d'une accélération du moteur,
- des capteurs de prise de mouvement,
- des capteurs d'accélération,
- des capteurs de force pour la détermination de forces transférées par les éléments de prothèse individuels à un environnement et pour la détermination de forces transférées par un environnement aux éléments de prothèse individuels et pour la détermination de forces interactives entre la prothèse et les supports de prothèse,
- des capteurs de couple pour la détermination des couples transférés par les éléments de prothèse individuels à un environnement et pour déterminer les couples transférés par un environnement aux éléments de prothèse individuels,
- des capteurs tactiles, en particulier une peau artificielle, pour la détection locale de forces et de couples actifs,
- des capteurs de température pour détecter des températures agissant sur la prothèse,
- des capteurs d'humidité pour détecter une humidité agissant sur la prothèse.

7. Prothèse selon une des revendications 1 à 6,
dans laquelle l'unité de commande (107), pour la commande de la prothèse, met à disposition plusieurs applications, c'est-à-dire des programmes et des jeux de paramètres de commande, chaque application définissant un mode de fonctionnement de la prothèse, et l'unité de commande présente une interface, en particulier une interface sans fil, avec une unité mobile, grâce à laquelle interface une application respective de commande de la prothèse peut être sélectionnée et configurée.

8. Prothèse selon une des revendications 1 à 7,
dans laquelle les deuxièmes capteurs (103) comprennent un ou plusieurs capteurs d'électromyographie et/ou plusieurs capteurs d'électroencéphalographie, les capteurs individuels pouvant être appliqués sur l'être vivant ou implantés dans l'être vivant.

9. Prothèse selon une des revendications 1 à 8,
dans laquelle l'unité de commande (107) est réalisée et conçue pour que, sur la base de l'état actuel ZUS(t) et des données D_{UMG}(t), il y ait une adaptation automatique de l'impédance mécanique active ou passive, en particulier de la rigidité de la prothèse, par une modification correspondante des signaux de commande Sig(t).

10. Prothèse selon une des revendications 1 à 9,
dans laquelle l'unité prédictive (105) est réalisée et conçue de manière à ce que la détermination du modèle M_{A}(t)/M_{A}(t) soit mise en œuvre sous forme d'un processus pouvant être appris qui, en se basant de manière autonome sur des données historiques de réalisation d'une action A/A', apprenne pour ce faire des mouvements prédits B_{eweg} (M_{A}(t) /B_{eweg} (M_{A'}(t) ) des éléments de la prothèse.
